# EUROPEAN PATENT APPLICATION

(11) **EP 4 775 982 A1**
(43) Date of publication of application: **15.07.2026**
(21) Application number: 24862855.4
(22) Date of filing: 05.09.2024
(51) Int. Cl.: G01N 33/02

(54) **FOOD PRODUCT EVALUATION DEVICE, FOOD PRODUCT EVALUATION METHOD, AND FOOD PRODUCT EVALUATION PROGRAM**

(30) Priority: 06.09.2023 JP 2023144838
(71) Applicant: AJINOMOTO CO., INC., Chuo-ku Tokyo 104-8315 (JP)
(72) Inventor: KIKUCHI, Takuya, Kawasaki-shi, Kanagawa 210-8681 (JP); TAKESHITA, Masamichi, Kawasaki-shi, Kanagawa 210-8681 (JP)
(74) Representative: Mewburn Ellis LLP
(86) International application number: PCT/JP2024/031822
(87) International publication number: WO 2025/053203

(57) **Abstract**

A protein is set as an evaluation target, and a digestibility prediction result on protein digestibility of the evaluation target is obtained using a machine learning model in which an amino acid composition of protein is an explanatory variable and protein digestibility is an objective variable.

## Description

### TECHNICAL FIELD

The present invention relates to a food evaluating apparatus, a food evaluating method, and a food evaluating program.

### BACKGROUND ART

Nonpatent Literature 1 discloses a technology to evaluate digestible and available amino acid contents using a digestible indispensable amino acid score (DIAAS), which is an amino acid score proposed by Food and Agriculture Organization of the United Nations (FAO) in 2013 to evaluate the balance of indispensable amino acids in protein materials and related products, and is the product of pig ileal digestibility data (standard ileal digestibility or true ileal digestibility) obtained from in vivo testing, the indispensable amino acid content of an object to be evaluated, and digestibility.

Nonpatent Literature 1: Dietary protein quality evaluation in human nutrition. Report of an FAO Expert Consultation, FAO Food Nutrition Paper, 2013; 92:1-66.

### DISCLOSURE OF INVENTION

### PROBLEM TO BE SOLVED BY THE INVENTION

However, in conventional inventions, digestibility data of an object to be evaluated is obtained by conducting in vivo testing, which is difficult in terms of animal ethics, testing costs, and low high-throughput.

The present invention is made in view of the problem, and an object of the present invention is to provide a food evaluating apparatus, a food evaluating method, and a food evaluating program that can predict digestibility using a protein material to be evaluated, then evaluate a digestible amino acid content obtained by multiplying the content of each amino acid by the digestibility, and provide a utilization method for the evaluation result.

### MEANS FOR SOLVING PROBLEM

In order to solve the above problem and attain this object, the food evaluating apparatus according to one aspect of the present disclosure is the food evaluating apparatus including a storage unit, and a control unit, wherein the storage unit includes a protein storage unit that stores a machine learning model in which an amino acid composition of protein is an explanatory variable and protein digestibility is an objective variable, and the control unit includes an evaluation target setting unit that sets the protein as an evaluation target, and a prediction result obtaining unit that obtains a digestibility prediction result on the protein digestibility of the evaluation target, using the machine learning model.

The food evaluating apparatus according to another aspect of the present disclosure is the food evaluating apparatus, wherein the explanatory variable includes a type of the protein.

The food evaluating apparatus according to still another aspect of the present disclosure is the food evaluating apparatus, wherein the type of the protein is a taxonomic rank of family, genus, or species of an animal or a plant.

The food evaluating apparatus according to still another aspect of the present disclosure is the food evaluating apparatus, wherein the explanatory variable further includes at least one of type of a processing and cooking method, temperature, and heating time.

The food evaluating apparatus according to still another aspect of the present disclosure is the food evaluating apparatus, wherein the explanatory variable further includes any one, some, or all of formulation data including any one or both of moisture content and hardness, physical-chemical property including any one or both of solubility and emulsifying power, nutrient content including any one, some, or all of dietary fiber content, fat content, carbohydrate content, and mineral content, part data of any one or both of a plant and an animal, and source data of any one, some, or all of country, region, and manufacturer.

The food evaluating apparatus according to still another aspect of the present disclosure is the food evaluating apparatus, wherein the protein is a protein as a cooking material or a protein contained in a single dish.

The food evaluating apparatus according to still another aspect of the present disclosure is the food evaluating apparatus, wherein the prediction result obtaining unit further calculates a digestible amino acid content using the digestibility prediction result, the digestible amino acid content being a value obtained by multiplying a content of each amino acid by the protein digestibility.

The food evaluating apparatus according to still another aspect of the present disclosure is the food evaluating apparatus, wherein the prediction result obtaining unit further calculates a digestible indispensable amino acid content, a digestible indispensable amino acid reference ratio, or a digestible indispensable amino acid score, using the digestibility prediction result, the digestible indispensable amino acid content being a value obtained by multiplying a content of each indispensable amino acid by the protein digestibility, the digestible indispensable amino acid reference ratio being a value obtained by dividing the digestible indispensable amino acid content by a reference value, the digestible indispensable amino acid score being a lowest value of the digestible indispensable amino acid content reference ratio.

The food evaluating apparatus according to still another aspect of the present disclosure is the food evaluating apparatus, wherein the control unit further includes a correcting unit that corrects the digestibility prediction result on the protein digestibility of protein contained in the single dish as the evaluation target, based on a digestibility change due to a combination of food ingredients that constitute the single dish.

The food evaluating apparatus according to still another aspect of the present disclosure is the food evaluating apparatus, wherein the control unit further includes a correcting unit that corrects the digestibility prediction result on the protein digestibility of protein contained in the single dish as the evaluation target, based on a digestibility change due to a combination with component dishes that constitute a menu that includes the single dish.

The food evaluating apparatus according to still another aspect of the present disclosure is the food evaluating apparatus, wherein the control unit further includes a correcting unit that corrects the digestibility prediction result on the protein digestibility of the evaluation target, based on a digestibility change due to exercise before and after intake of the evaluation target.

The food evaluating apparatus according to still another aspect of the present disclosure is the food evaluating apparatus, wherein the control unit further includes a correcting unit that corrects the digestibility prediction result on the protein digestibility of the evaluation target, based on a digestibility change due to a constitution of a human who consumes the evaluation target.

The food evaluating apparatus according to still another aspect of the present disclosure is the food evaluating apparatus, wherein the prediction result obtaining unit further obtains dietary suggestion data, based on the digestibility prediction result and nutrient data including a dietary reference intake.

The food evaluating apparatus according to still another aspect of the present disclosure is the food evaluating apparatus, wherein the prediction result obtaining unit further displays the digestibility prediction result.

The food evaluating apparatus according to still another aspect of the present disclosure is the food evaluating apparatus, wherein the evaluation target is the protein consumed over a predetermined period of time for one or more meals.

The food evaluating apparatus according to still another aspect of the present disclosure is the food evaluating apparatus, wherein the prediction result obtaining unit obtains the dietary suggestion data, based on the digestibility prediction result, and the nutrient data including the dietary reference intake according to any one or both of an attribute and an intake purpose of a human who consumes the evaluation target.

The food evaluating apparatus according to still another aspect of the present disclosure is the food evaluating apparatus, wherein the prediction result obtaining unit further creates product development data for a product that reflects the protein digestibility of the evaluation target, based on the digestibility prediction result.

The food evaluating apparatus according to still another aspect of the present disclosure is the food evaluating apparatus, wherein the machine learning model is a model including at least one of random forests, neural networks, XGBoost, LightGBM, support vector regression, and linear regression including ElasticNet.

The food evaluating apparatus according to still another aspect of the present disclosure is the food evaluating apparatus including a storage unit, and a control unit, wherein the storage unit includes a protein storage unit that stores a machine learning model in which an amino acid composition of protein is an explanatory variable, and a digestible amino acid content, a digestible indispensable amino acid content, a digestible indispensable amino acid reference ratio, or a digestible indispensable amino acid score is an objective variable, and the control unit includes an evaluation target setting unit that sets the protein as an evaluation target, and a prediction result obtaining unit that obtains the digestible amino acid content, the digestible indispensable amino acid content, the digestible indispensable amino acid reference ratio, or the digestible indispensable amino acid score of the evaluation target, using the machine learning model.

The food evaluating apparatus according to still another aspect of the present disclosure is the food evaluating apparatus including a storage unit, and a control unit, wherein the storage unit includes a protein storage unit that stores, as training data, a data set in which at least one of an amino acid composition of protein, a type of the protein, formulation data including any one or both of water content and hardness, physical-chemical property including any one or both of solubility and emulsifying power, a nutrient content including any one, some, or all of dietary fiber content, fat content, carbohydrate content, and mineral content, part data of any one or both of a plant and an animal, source data of any one, some, or all of country, region, and manufacturer, a type of a processing and cooking method, a cooking temperature, and a cooking time, and an in vitro analysis result on any one or both of enzyme activity inhibition and digestibility is an explanatory variable, and protein digestibility in an in vitro process or an in vivo process is an objective variable, and the control unit includes a model constructing unit that trains a machine learning model with the training data.

The food evaluating method according to still another aspect of the present disclosure is the food evaluating method executed by a food evaluating apparatus including a storage unit, and a control unit, wherein the storage unit includes a protein storage unit that stores a machine learning model in which an amino acid composition of protein is an explanatory variable and protein digestibility is an objective variable, the method executed by the control unit including an evaluation target setting step of setting the protein as an evaluation target, and a prediction result obtaining step of obtaining a digestibility prediction result on the protein digestibility of the evaluation target, using the machine learning model.

The food evaluating method according to still another aspect of the present disclosure is the food evaluating method executed by a food evaluating apparatus including a storage unit, and a control unit, wherein the storage unit includes a protein storage unit that stores, as training data, a data set in which at least one of an amino acid composition of protein, a type of the protein, formulation data including any one or both of water content and hardness, physical-chemical property including any one or both of solubility and emulsifying power, a nutrient content including any one, some, or all of dietary fiber content, fat content, carbohydrate content, and mineral content, part data of any one or both of a plant and an animal, source data of any one, some, or all of country, region, and manufacturer, a type of a processing and cooking method, a cooking temperature, and a cooking time, and an in vitro analysis result on any one or both of enzyme activity inhibition and digestibility is an explanatory variable, and protein digestibility in an in vitro process or an in vivo process is an objective variable, the method executed by the control unit including a model constructing step of training a machine learning model with the training data.

The food evaluating program according to still another aspect of the present disclosure is the food evaluating program including programmed instructions for causing, when executed by a food evaluating apparatus including a storage unit that includes a protein storage unit that stores a machine learning model in which an amino acid composition of protein is an explanatory variable and protein digestibility is an objective variable, and a control unit, the control unit of the food evaluating apparatus to perform a food evaluating method including an evaluation target setting step of setting the protein as an evaluation target, and a prediction result obtaining step of obtaining a digestibility prediction result on the protein digestibility of the evaluation target, using the machine learning model.

### EFFECT OF THE INVENTION

According to the present invention, digestibility can be predicted while incorporating nutrient information on food ingredients, ash, moisture content, and the like other than protein. According to the present invention, a menu that maximizes the use of vegetable protein while accounting for DIAAS is proposed and thereby being able to be applied to solving health issues of the elderly, athletes, diseases, and reduced digestibility due to combinations of foods. According to the present invention, DIAAS can be estimated for food ingredients (including vegetable protein) subjected to various processing such as cooking and processing, thereby being able to contribute to the development of high "quality" protein products. According to the present invention, DIAAS can be estimated on a product level.

### BRIEF DESCRIPTION OF DRAWINGS

FIG. 1 is a block diagram of an example of the configuration of a food evaluating apparatus according to an embodiment of the present invention;
FIG. 2 is a flowchart of an example of food evaluation processing according to the embodiment;
FIG. 3 is a diagram of an example of data collection according to the embodiment;
FIG. 4 is a diagram of an example of model construction according to the embodiment;
FIG. 5 is a chart of an example of protein digestibility prediction according to the embodiment;
FIG. 6 is a diagram of an example of digestible indispensable amino acid reference ratio prediction according to the embodiment;
FIG. 7 is a diagram of an example of differences between evaluating methods according to the embodiment;
FIG. 8 is a diagram of an example of assumptions in evaluating methods according to the embodiment;
FIG. 9 is a table of an example of biological species for digestibility data according to the embodiment;
FIG. 10 is a table of an example of protein type major categories according to the embodiment;
FIG. 11 is a table of an example of protein type subcategories according to the embodiment;
FIG. 12 is a table of an example of types of cooking and processing methods according to the embodiment;
FIG. 13 is a chart of an example of protein digestibility prediction according to the embodiment;
FIG. 14 is a table of an example of coefficients of determination according to the embodiment;
FIG. 15 is a table of an example of the coefficients of determination according to the embodiment;
FIG. 16 is a table of an example of the coefficients of determination according to the embodiment;
FIG. 17 is a table of an example of the coefficients of determination according to the embodiment;
FIG. 18 is a chart of an example of digestible indispensable amino acid reference ratio according to the embodiment;
FIG. 19 is a chart of an example of prediction accuracy verification results according to the embodiment;
FIG. 20 is a chart of an example of prediction accuracy verification results according to the embodiment;
FIG. 21 is a chart of an example of prediction accuracy verification results according to the embodiment;
FIG. 22 is a table of an example of prediction accuracy verification results according to the embodiment;
FIG. 23 is a chart of an example of explanatory variable importance analysis results according to the embodiment;
FIG. 24 is a chart of an example of the explanatory variable importance analysis results according to the embodiment;
FIG. 25 is a chart of an example of correlation according to the embodiment;
FIG. 26 is a chart of an example of correlation according to the embodiment; and
FIG. 27 is a chart of an example of correlation according to the embodiment.

### BEST MODE(S) FOR CARRYING OUT THE INVENTION

Exemplary embodiments of the present invention will be explained in detail with reference to the drawings. The present invention is not limited by the embodiments.

### 1. Overview

An overview of the present invention will be explained.

Conventionally, there are multiple amino acid scores. DIAAS is a newer score, established by the FAO in 2013. DIAAS is an amino acid score that accounts for the recommended daily intake of amino acid and the digestibility of protein. DIAAS is the lowest value of digestible indispensable amino acid reference ratios of each indispensable amino acid. The digestible indispensable amino acid reference ratio (DIAA) is calculated by "digestible indispensable amino acid content/reference value". The digestible indispensable amino acid content is calculated by "content of each indispensable amino acid (mg/g_protein)×digestibility (%)". The reference value is defined based on a good amino acid balance (as described in FAO DIAAS guidelines). DIAAS changes depending on the type of food ingredient, processing (e.g., freezing and thawing), or cooking (e.g., grilling). The digestible indispensable amino acid content is calculated by "(content of each indispensable amino acid (mg/g_protein)×digestibility (%))", and the digestible amino acid content is calculated by "(content of each amino acid (mg/g_protein)×digestibility (%))".

Conventionally, DIAAS calculation requires three items: (1) the amino acid content in protein, (2) the recommended intake of amino acid provided by FAO, and (3) the in vivo digestibility of protein, which needs to be measured in vivo (e.g., pigs). The evaluation is generally difficult due to lack of data for (3). Therefore, conventionally, promoting high DIAAS is merely focused on animal proteins, based on the known DIAAS evaluation results.

The present embodiment provides a scheme to enable evaluation and prediction of DIAAS for unknown conditions that are not evaluated in vivo, by predicting protein digestibility with an in silico prediction model.

In other words, an object of the present embodiment is to provide a method for evaluating DIAAS of materials or products or evaluating digestible indispensable amino acid contents in dietary records, as well as a method for utilizing the evaluation result, after predicting digestibility using the type of protein material to be evaluated (e.g., genus or family of vegetable protein), a cooking/processing method (type (e.g., fermented, boiled, or frozen) and quantitative data (e.g., boiling time, heating temperature, or grind size)), and amino acid composition. For the amino acid composition and the digestibility data, the measurement results of 18 amino acids (Arg, His, Ile, Leu, Lys, Met, Phe, Thr, Trp, Val, Ala, Asp, Cys, Glu, Gly, Ser, Tyr, Pro) are used. For the digestibility data, the results of in vivo testing (e.g., human, pig, or rodent) and artificial intestinal juice testing (in vitro results) are used.

The prediction technique according to the present embodiment is characterized by construction of a prediction model by machine learning (any one, some, or all of random forest, neural network, XGBoost, LightGBM, support vector regression, and linear regression including ElasticNet, etc.) using the digestibility data as an objective variable, and the amino acid composition, the cooking/processing method, and the type of protein as explanatory variables. In the method for evaluating DIAAS of materials or products or evaluating the digestible amino acid contents in dietary records, the ratio of the predicted amount of amino acid utilization to the recommended amount of amino acid utilization as proposed by FAO is compared with the ratio in a protein material as a reference, based on the predicted digestibility data.

### 2. Configuration of Food Evaluating Apparatus 100

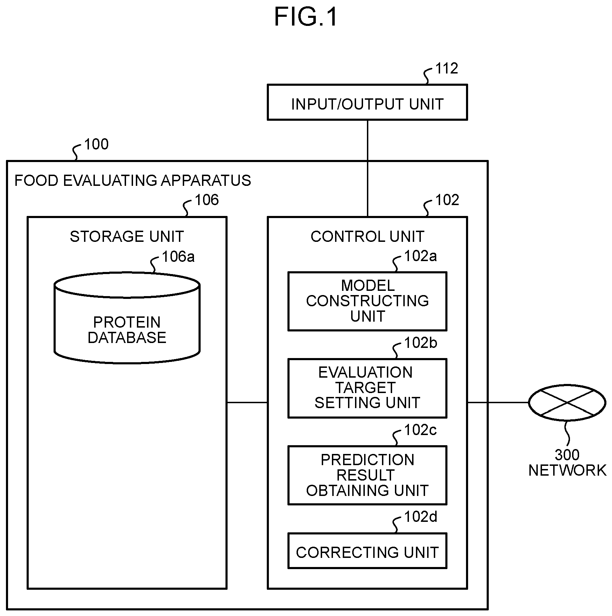

A food evaluating apparatus 100 according to the present embodiment can be configured to be functionally or physically distributed or integrated in any units (either stand-alone type or system type). In the present embodiment, an example of the configuration of the food evaluating apparatus 100 will be explained with reference to FIG. 1. FIG. 1 is a block diagram of an example of the configuration of the food evaluating apparatus 100 according to the present embodiment.

As shown in FIG. 1, the food evaluating apparatus 100 may be an information processing apparatus such as a personal computer or a workstation. The food evaluating apparatus 100 includes a control unit 102, a storage unit 106, and an input/output unit 112. The units of the food evaluating apparatus 100 are communicatively connected via any communication channel. The food evaluating apparatus 100 is mutually communicatively connected to other devices via a network 300.

The input/output unit 112 may have a function to perform data input/output (I/O). The input/output unit 112 may be, for example, a key input unit, a touch panel, a control pad (e.g., touch pad and game pad), a mouse, a keyboard, and a microphone. The input/output unit 112 may be a display unit (e.g., a display, a monitor, and a touch panel configured with liquid crystal or organic EL) that displays (input/output) information of application software and the like. The input/output unit 112 may be an audio output unit (e.g., speaker) that outputs audio information as sound and voice. The input/output unit 112 may be an image input unit (e.g., camera) that records images (still images and moving images) captured by an imaging device such as a CCD image sensor or a CMOS image sensor as digital data. The input/output unit 112 may be any one, some, or all of a fingerprint sensor, a camera (e.g., infrared camera) that can be used for iris recognition or face recognition or the like, and a biometric sensor such as a vein sensor.

The storage unit 106 stores any one, some, or all of various databases, tables, and files and the like. The storage unit 106 has a computer program recorded for performing various processing by giving instructions to a central processing unit (CPU) in cooperation with an operating system (OS). For example, any one, some, or all of a random access memory (RAM), a read only memory (ROM), a hard disk drive (HDD), and a solid state drive (SSD) can be used as the storage unit 106. The storage unit 106 may store any one, some, or all of image data recorded at the input/output unit 112, data received via the network 300, and input data entered via the input/output unit 112 and the like. The storage unit 106 functionally and conceptually includes a protein database 106a.

The protein database 106a stores protein data. The protein database 106a may store a machine learning model pertaining to proteins. The protein database 106a may store a machine learning model in which the amino acid composition of protein is an explanatory variable and the digestibility of protein (protein digestibility) is an objective variable. The explanatory variable may include the type of protein. The type of protein may be a taxonomic rank of family, genus, or species of an animal or a plant. The explanatory variable may include at least one of type of a processing and cooking method, temperature, and heating time. The explanatory variable may include any one, some, or all of formulation data including any one or both of moisture content and hardness, physical-chemical property including any one or both of solubility and emulsifying power, nutrient content including any one, some, or all of dietary fiber content, fat content, carbohydrate content, and mineral content, part data of any one or both of a plant and an animal, and source data of any one, some, or all of country, region, and manufacturer. The protein may be a protein as a cooking material or a protein contained in a single dish. The machine learning model may be a model including at least one of random forests, neural networks, XGBoost, LightGBM, support vector regression, and linear regression including ElasticNet. The protein database 106a may store a data set (variable data) serving as explanatory and objective variables for machine learning. The protein database 106a may store a machine learning model in which the amino acid composition of protein is an explanatory variable, and the digestible amino acid content, the digestible indispensable amino acid content, the digestible indispensable amino acid reference ratio, or the digestible indispensable amino acid score is an objective variable. The protein database 106a may store, as training data, a data set in which at least one of an amino acid composition of protein, a type of the protein, formulation data including any one or both of water content and hardness, physical-chemical property including any one or both of solubility and emulsifying power, a nutrient content including at least one, some, or all of dietary fiber content, fat content, carbohydrate content, and mineral content, part data of any one or both of a plant and an animal, source data of at least one, some, or all of country, region, and manufacturer, a type of a processing and cooking method, a cooking temperature, and a cooking time, and an in vitro analysis result on any one or both of enzyme activity inhibition and digestibility is an explanatory variable, and protein digestibility in an in vitro process or an in vivo process is an objective variable.

The control unit 102 is a CPU or the like that overall controls the food evaluating apparatus 100. The control unit 102 has an internal memory for storing a control program such as an OS, a computer program that specifies various processing procedures, required data, and the like, and executes various information processing based on these stored computer programs. The control unit 102 functionally and conceptually includes a model constructing unit 102a, an evaluation target setting unit 102b, a prediction result obtaining unit 102c, and a correcting unit 102d.

The model constructing unit 102a constructs a machine learning model. The model constructing unit 102a may train the machine learning model with training data. The model constructing unit 102a may register a data set serving as explanatory and objective variables for machine learning in the protein database 106a.

The evaluation target setting unit 102b sets a protein as an evaluation target. The evaluation target may be a protein consumed over a predetermined period of time for one or more meals.

The prediction result obtaining unit 102c obtains a digestibility prediction result on protein digestibility of protein. The prediction result obtaining unit 102c may obtain the digestibility prediction result on protein digestibility of the evaluation target, using the machine learning model. The prediction result obtaining unit 102c may calculate the digestible amino acid content, which is a value obtained by multiplying the content of each amino acid by the protein digestibility, using the digestibility prediction result. The prediction result obtaining unit 102c may calculate the digestible indispensable amino acid content, which is a value obtained by multiplying the content of each indispensable amino acid by the protein digestibility, the digestible indispensable amino acid reference ratio, which is a value obtained by dividing the digestible indispensable amino acid content by a reference value, or the digestible indispensable amino acid score, which is the lowest value of the digestible indispensable amino acid reference ratio, using the digestibility prediction result. The prediction result obtaining unit 102c may obtain dietary suggestion data, based on the digestibility prediction result and nutrient data including a dietary reference intake. The prediction result obtaining unit 102c may display the digestibility prediction result. The prediction result obtaining unit 102c may obtain dietary suggestion data, based on the digestibility prediction result, and nutrient data including any one or both of a dietary reference intake according to the attribute and an intake purpose of a human who consumes the evaluation target. The prediction result obtaining unit 102c may create product development data for a product that reflects the protein digestibility of the evaluation target, based on the digestibility prediction result. The prediction result obtaining unit 102c may obtain the digestible amino acid content, the digestible indispensable amino acid content, the digestible indispensable amino acid reference ratio, or the digestible indispensable amino acid score of the evaluation target, using the machine learning model.

The correcting unit 102d corrects the digestibility prediction result. The correcting unit 102d may correct the digestibility prediction result on protein digestibility of protein contained in a single dish as the evaluation target, based on a digestibility change due to a combination with component dishes that constitute a menu that includes the single dish. The correcting unit 102d may correct the digestibility prediction result on protein digestibility of the evaluation target, based on a digestibility change due to exercise before and after intake of the evaluation target. The correcting unit 102d may correct the digestibility prediction result on protein digestibility of the evaluation target, based on a digestibility change due to the constitution of a human who consumes the evaluation target. The correcting unit 102d may correct the digestibility prediction result on protein digestibility of protein contained in a single dish as the evaluation target, based on a digestibility change due to a combination of food ingredients that constitute the single dish.

### 3. Food Evaluation Processing

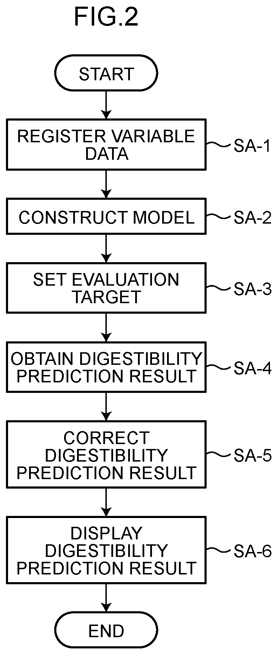

An example of food evaluation processing according to the present embodiment will be explained with reference to FIG. 2 to FIG. 10. FIG. 2 is a flowchart of an example of the food evaluation processing according to the present embodiment.

As depicted in FIG. 2, when a data set serving as explanatory and objective variables for machine learning is input by a user via the input/output unit 112, the model constructing unit 102a registers the data set as training data in the protein database 106a (step SA-1).

The model constructing unit 102a constructs a machine learning model in which at least the amino acid composition of protein is an explanatory variable and the protein digestibility is an objective variable, based on the variable data stored in the protein database 106a (step SA-2).

If a protein is specified by the user via the input/output unit 112, the evaluation target setting unit 102b sets the protein as the evaluation target (step SA-3).

The prediction result obtaining unit 102c obtains the digestibility prediction result on protein digestibility of the evaluation target, using the machine learning model stored in the protein database 106a, and calculates any one, some, or all of the digestible amino acid content, the digestible indispensable amino acid content, the digestible indispensable amino acid reference ratio, and the digestible indispensable amino acid score, using the digestibility prediction result (step SA-4).

The correcting unit 102d corrects the digestibility prediction result on protein digestibility of the evaluation target, based on a digestibility change due to any one or both of exercise before and after intake of the evaluation target and a digestibility change due to the constitution of a human who consumes the evaluation target and the like (step SA-5).

The prediction result obtaining unit 102c displays the digestibility prediction result on the input/output unit 112 (step SA-6) and terminates the processing.

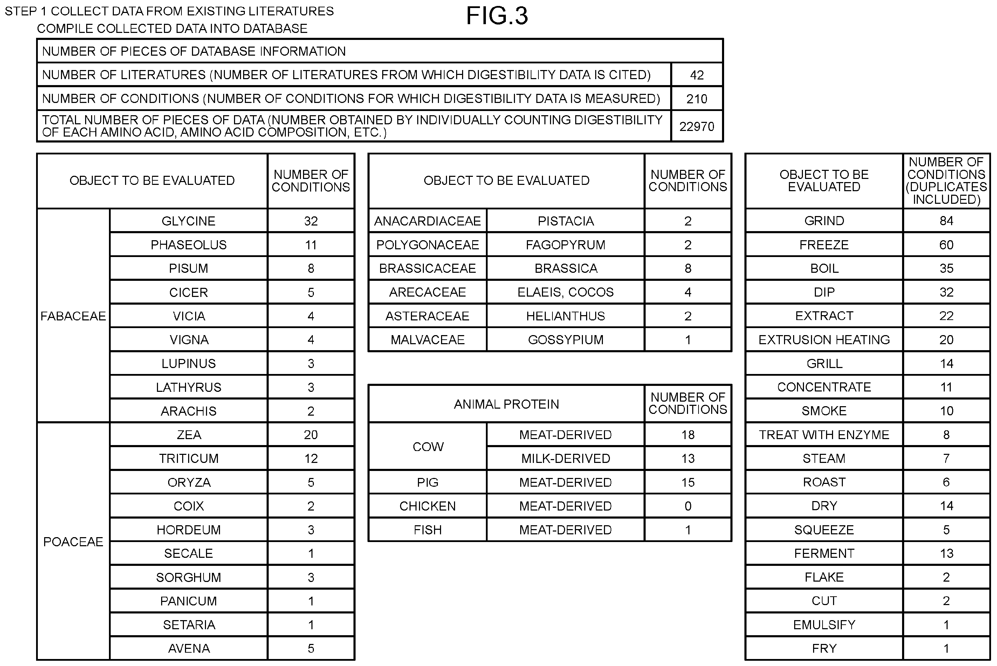

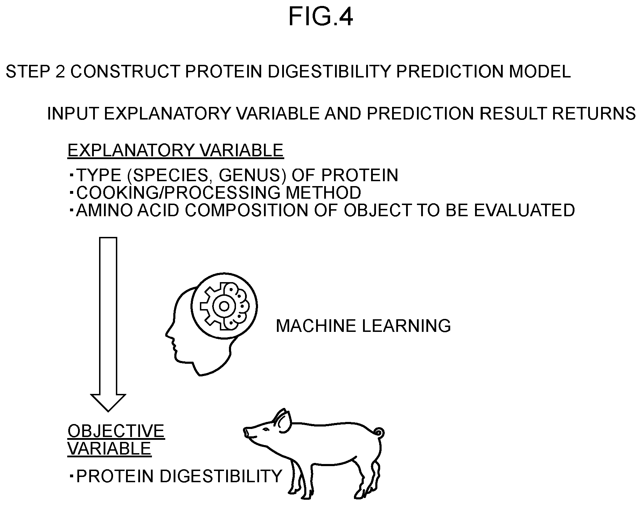

Referring to FIG. 3 and FIG. 4, an example of model construction processing according to the present embodiment will be explained. FIG. 3 is a diagram of an example of data collection according to the present embodiment. FIG. 4 is a diagram of an example of model construction according to the present embodiment.

As depicted in FIG. 3, according to the present embodiment, the type of protein (species, genus), the cooking/processing method, the amino acid composition of an object to be evaluated, and the protein digestibility data are collected from existing literatures and compiled into a database (STEP 1).

As depicted in FIG. 4, according to the present embodiment, a protein digestibility prediction model is constructed to perform machine learning using the protein digestibility as an objective variable and other data as explanatory variables (STEP 2). The protein digestibility prediction model does not necessarily require the amino acid composition data as input data, and the prediction may be based on digestibility changes due to a food combination, aging, exercise, and the like.

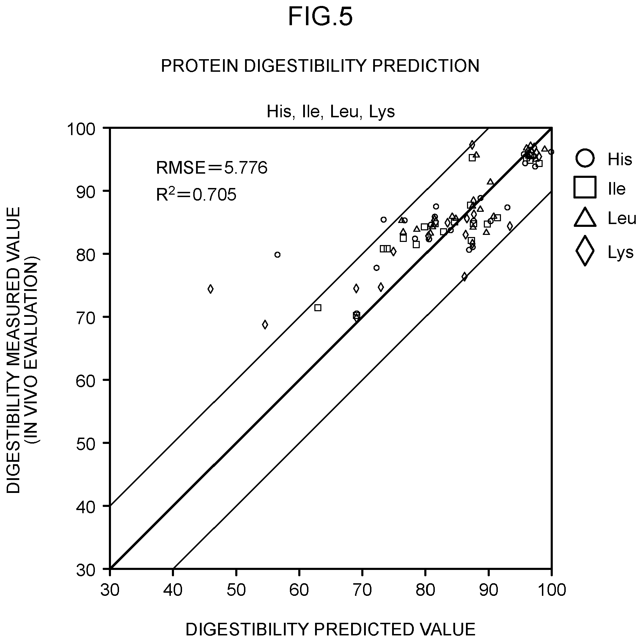

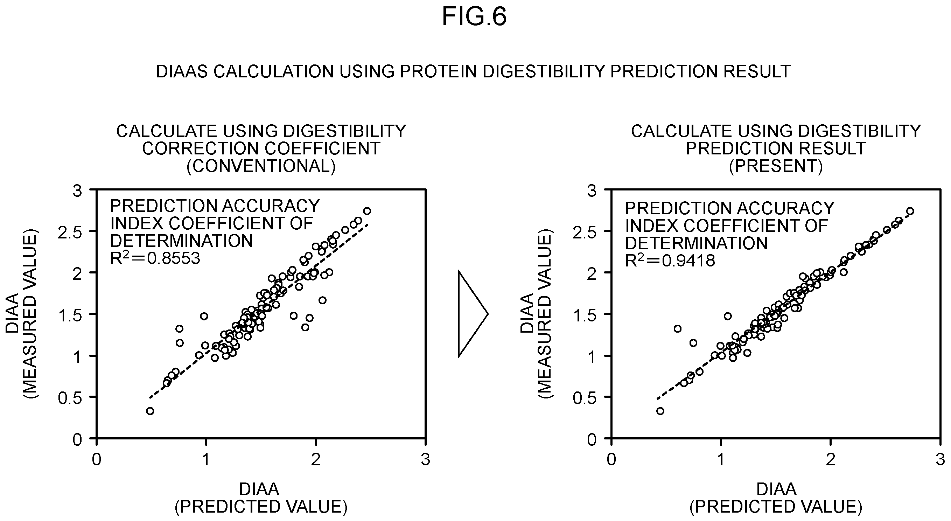

Referring to FIG. 5 and FIG. 6, an example of prediction accuracy verification according to the present embodiment will be explained. FIG. 5 is a chart of an example of protein digestibility prediction according to the present embodiment. FIG. 6 is a diagram of an example of digestible indispensable amino acid reference ratio prediction according to the present embodiment.

As depicted in FIG. 5, according to the present embodiment, in 96 out of 100 conditions, errors from the measured values are within 10% as indicated by the plot of protein digestibility results (100 predicted values) for four amino acids for 25 proteins.

As depicted in FIG. 6, according to the present embodiment, a comparison of the correlation of the digestible indispensable amino acid reference ratio (conventional) calculated using a correction coefficient for protein digestibility (average of all conditions (86.4%)) and the digestible indispensable amino acid reference ratio (present) calculated using the digestibility prediction result with the actually measured values indicates that the predictability of the digestible indispensable amino acid reference ratio is improved by using the protein digestibility model.

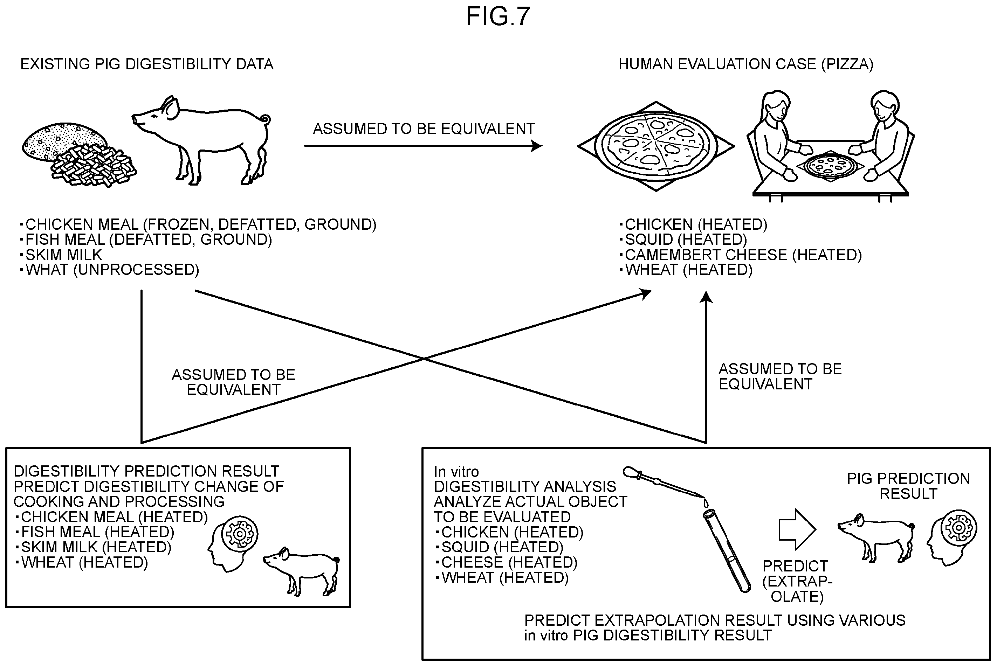

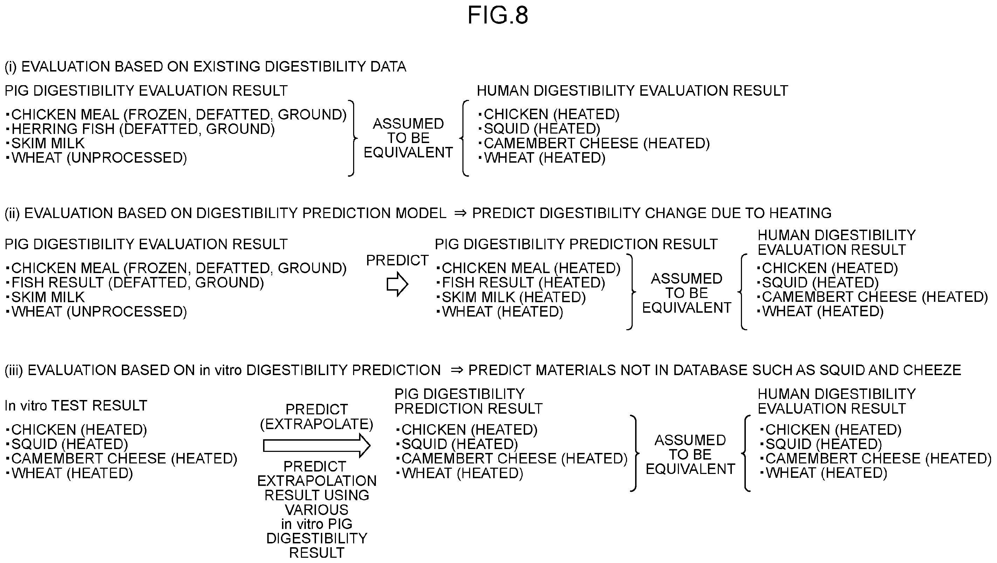

Referring to FIG. 7 and FIG. 8, an example of evaluating methods according to the present embodiment will be explained. FIG. 7 is a diagram of an example of differences between evaluating methods according to the present embodiment. FIG. 8 is a diagram of an example of assumptions in evaluating methods according to the present embodiment.

As shown in FIG. 7, according to the present embodiment, assuming that pig protein digestibility is equivalent to human protein digestibility, although there may be errors in prediction accuracy due to the differences in cooking and processing methods and materials, human protein digestibility prediction is performed using existing pig digestibility data, and human extrapolation results are predicted using various in vitro pig digestibility results.

As depicted in FIG. 8, according to the present embodiment, (i) in the evaluation based on existing digestibility data, it is assumed that the pig digestibility evaluation result is equivalent to the human digestibility evaluation result, although the cooking and processing methods and materials are different; (ii) in the evaluation based on the digestibility prediction model that predicts a digestibility change due to heating, it is assumed that the pig digestibility prediction result predicted from the pig digestibility evaluation result is equivalent to the human digestibility evaluation result, although the materials are different; and (iii) in the evaluation based on in vitro digestibility prediction, which also predicts materials not in the database, such as squid and cheese, it is assumed that the pig digestibility prediction result extrapolated from in vitro test results is equivalent to the human digestibility evaluation result.

According to the present embodiment, data on cooking/processing method may be excluded from the explanatory variables in the protein digestibility prediction model, and one or some of the types of amino acids to be added to the explanatory variables may be excluded. According to the present embodiment, to refine the digestibility prediction result, formulation data (water content, hardness, etc.), physical-chemical property (solubility, emulsifying power, etc.), nutrient content (dietary fiber, fat content, carbohydrates, minerals, etc.), protein material data (plant parts (bean, pod, stalk, flower, etc.), animal parts (loin, fillet, leg, etc.), and source (country, region, manufacturer, etc.)) of the object to be evaluated may be added to explanatory variables.

According to the present embodiment, the machine learning model may be used in such a manner that the data set of explanatory variables is sorted into a plurality of groups according to the purpose of evaluation and the contents of the training data set, and a plurality of models including a model for predicting digestibility and a model for correcting the digestibility prediction result are coupled to each other.

According to the present embodiment, in the prediction of digestibility data, the construction of the protein digestibility prediction model may be a method for obtaining a predicted value using a convenient factor such as a scaling factor based on evidence on digestibility changes analyzed in humans and animals, or a method for correcting the digestibility prediction result in machine learning based on evidence on digestibility changes. The evidence to be used may be a digestibility change due to a food combination (e.g., effects of digestive enzymes derived from materials or digestive enzyme inhibitors), a digestibility change due to exercise (e.g., type, duration, or frequency of exercise), or a digestibility change due to body constitution (e.g., change in digestive enzyme secretion or digestive tract function due to aging, disease, and the like, or effects of in vivo amino acid availability).

In the food evaluating method according to the present embodiment, the evaluation target may include food records for various periods of time, such as one meal, one day, or one month. According to the present embodiment, a value other than the reference value proposed by FAO may be used as the recommended amount of amino acid utilization as a reference value, in consideration of the attribute of a subject to be evaluated (e.g., athlete or person with disease) and the purpose of evaluation (e.g., dieting or beauty purpose).

The food evaluating method according to the present embodiment may include a function related to dietary suggestion, such as recipes and menus, based on data on nutrients, such as dietary reference intake. According to the present embodiment, the dietary suggestion may be performed based on data on a person who consumes food (e.g., mental data (preferences, mood, etc.), analysis results (body measurements (height, weight, energy expenditure, brain wave, etc.), biochemical test items (blood pressure, metabolites, etc.), genetic analysis (gene sequence and expression, gut bacteria, etc.)), and external data (e.g., seasons and trends).

In the food evaluating method according to the present embodiment, any one or both of the digestibility prediction and the results obtained from any one, some, or all of the digestible amino acid content, digestible indispensable amino acid content, digestible indispensable amino acid reference ratio, and digestible indispensable amino acid score evaluation may be visualized.

According to the present embodiment, it is possible to predict a digestibility change when a hypothetical cooking/processing method is performed for a material to be evaluated, and based on the digestibility prediction result, development and improvement of a cooking/processing method as well as product development (e.g., food product development) may be performed in which it is intended that the digestibility, the digestible amino acid content, the digestible indispensable amino acid content, the digestible indispensable amino acid reference ratio, or the digestible indispensable amino acid score of the material to be evaluated changes.

### Example 1

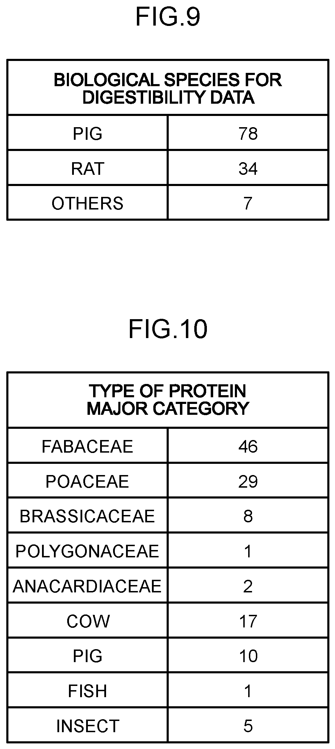

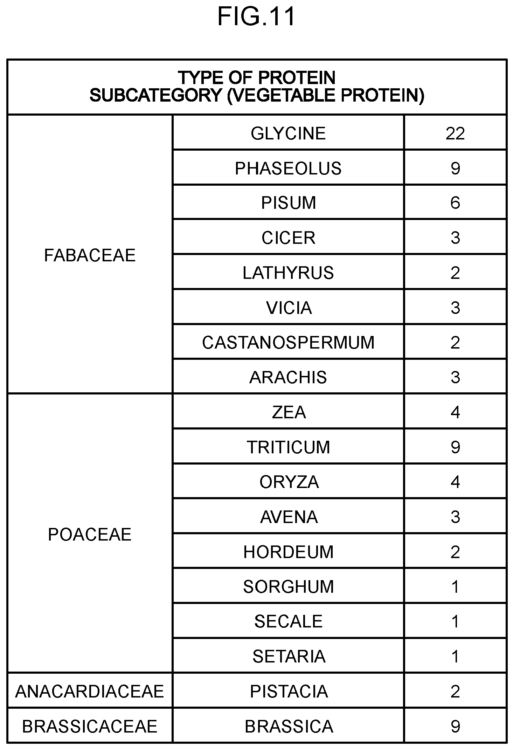

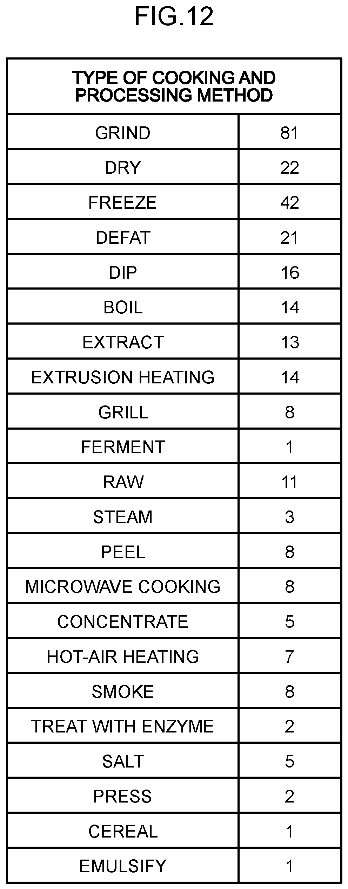

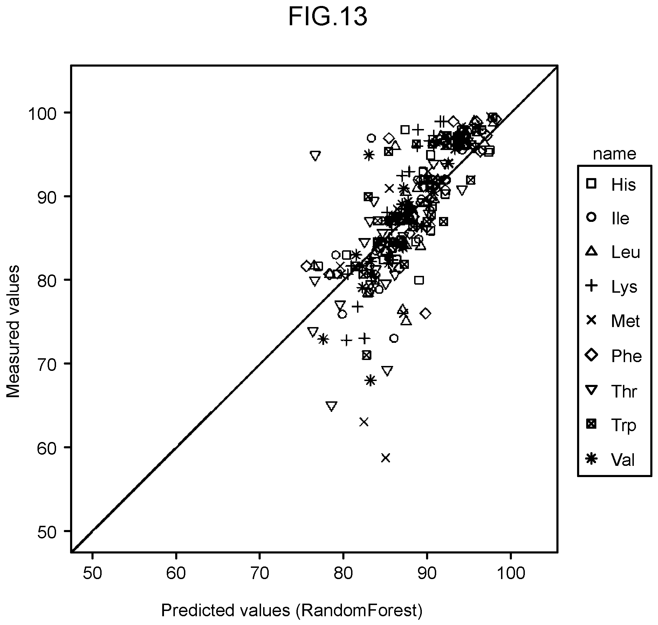

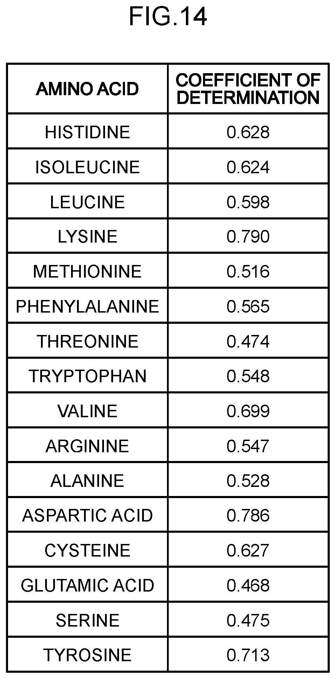

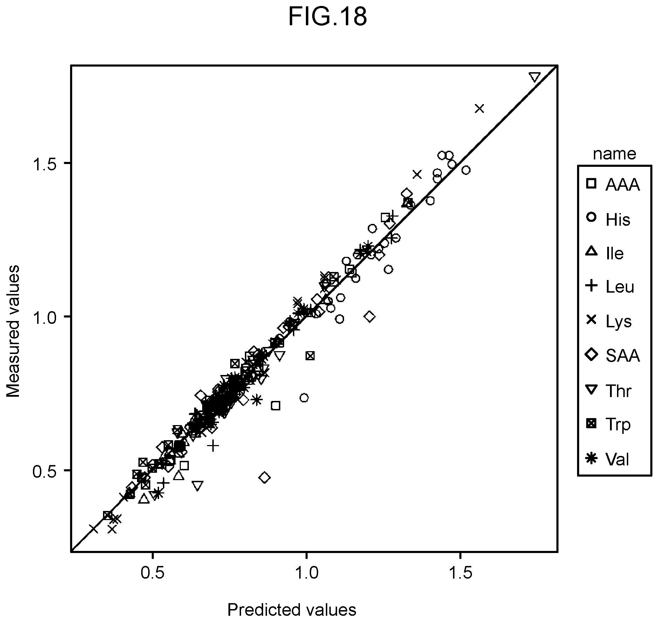

Referring to FIG. 9 to FIG. 18, Example 1 will be explained. FIG. 9 is a table of an example of biological species for digestibility data according to the present embodiment. FIG. 10 is a table of an example of protein type major categories according to the present embodiment. FIG. 11 is a table of an example of protein type subcategories according to the present embodiment. FIG. 12 is a table of an example of the types of cooking and processing methods according to the present embodiment. FIG. 13 and FIG. 18 are charts of examples of protein digestibility prediction according to the present embodiment. FIG. 14 to FIG. 17 are tables of examples of the coefficients of determination according to the present embodiment.

According to the present embodiment, digestibility prediction accuracy verification is performed for 119 conditions of digestibility test results for pigs, rats, and the like described in previous literatures.

### (1) Digestibility Data Collection

In this example, digestibility test results (119 conditions) in pigs, rats, and the like, DIAA, and amino acid compositions of materials to be evaluated that are listed on PubMed (http://www.ncbi.nlm.nih.gov/pubmed/) are collected, and the types of materials to be evaluated are classified into each item as indicated in FIG. 9 to FIG. 12.

### (2) Data Preprocessing

Some of the previously published literatures that list the digestibility and the amino acid compositions used in this example do not list DIAA, and the amino acid compositions are listed in different evaluation methods or units depending on whether the water content in the object to be evaluated is considered. In this example, defective DIAA is compensated for by calculation according to the guidance by FAO (35978-02317b979a686a57aa4593304ffc17f06.pdf (fao.org)). In this example, the amino acid compositions are converted into a form that can be uniformly used for machine learning, by calculation that aligns the amino acid compositions to a common unit across literatures.

### (3) Digestibility Prediction Model Construction

In this example, the programming language "R" is used to perform construction of a digestibility prediction random forest model. In this example, digestibility data (quantitative data) from pigs, rats, and the like are used as objective variables, and qualitative data classified according to the items depicted in FIG. 10 to FIG. 12 for the amino acid composition of the material to be evaluated (quantitative data) and the type of protein in the material to be evaluated are used as explanatory variables. The digestibility analysis results in this example are obtained from the amino acid analysis values of undigested substances in pig ileum, and therefore are obtained as individual values for each amino acid. In this example, 119 conditions randomly selected from among these data sets are used as training data and 28 conditions as evaluation data, and optimization of hyperparameters is performed by cross-validation based on 10 partitions of the training data.

### (4) Prediction Accuracy Verification of Digestibility Prediction Model

As depicted in FIG. 13, in this example, the comparison of the predicted digestibility data (Predicted values) and the measured values (Measured values) is plotted based solely on amino acid compositions. As depicted in FIG. 13, this example indicates that a precise prediction close to the straight line in the graph is made in many conditions, in other words, the predicted value and the measured value are close to each other. As depicted in FIG. 14, in this example, the coefficient of determination indicating the goodness of fit of regression analysis, which is one of indexes of prediction accuracy, is determined for the digestibility data predicted solely from the amino acid compositions and the actual measured values. In general, if the coefficient of determination is 0.5 or higher, the prediction accuracy is considered to be good to some extent, although it is only a relative reference. As depicted in FIG. 14, in this example, the coefficients of determination exceed 0.5 for 13 amino acids except threonine, glutamic acid, and serine, and in particular, the coefficients of determination for lysine and tyrosine exceed 0.7, indicating good predictability.

### (5) Refinement of Digestibility Prediction Model

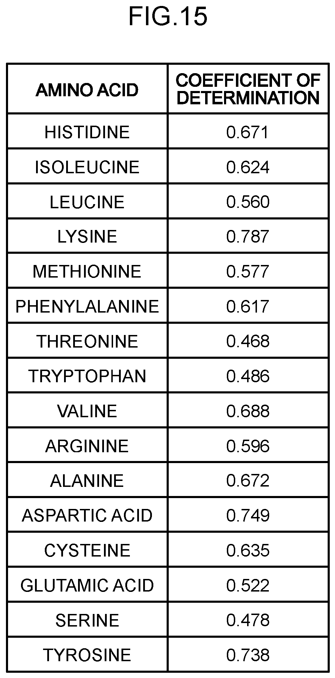

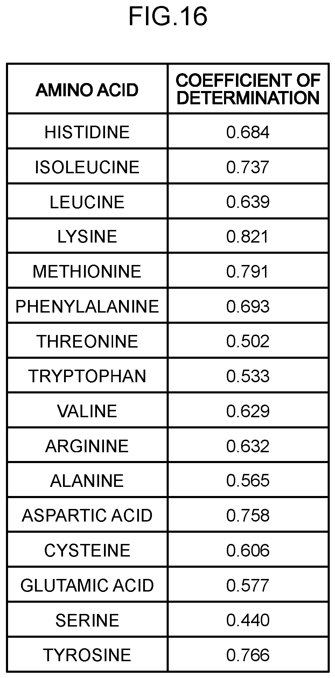

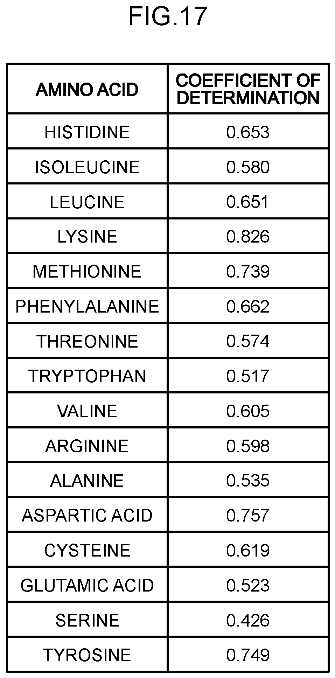

In this example, when the type of protein (qualitative data) is added to the explanatory variables in "(3) Digestibility Prediction Model Construction", the coefficients of determination depicted in FIG. 15 are obtained. When the type of protein (qualitative data) and the type of cooking and processing method (qualitative data) are added to the explanatory variables in "(3) Digestibility Prediction Model Construction", the coefficients of determination depicted in FIG. 16 are obtained. When the type of protein (qualitative data), the type of cooking and processing method (qualitative data), and time and temperature (quantitative data) are added to the explanatory variables in "(3) Digestibility Prediction Model Construction", the coefficients of determination depicted in FIG. 17 are obtained. As a result, as depicted in FIG. 16 and FIG. 17, in this example, adding explanatory variables reduces the number of amino acids with a coefficient of determination below 0.5 to one amino acid, serine, and adding data on both the type of protein and the type of cooking and processing method to the explanatory variables increases the number of amino acids with a coefficient of determination exceeding 0.7 to four or more. Based on the above, this example indicates that more precise predictions can be made by adding not only the amino acid composition but also the type of protein and the type of cooking and processing method to the explanatory variables.

In this example, using the digestibility results predicted using the digestibility prediction model (the only explanatory variable is amino acid composition) constructed in "(3) Digestibility Prediction Model Construction", evaluation of the digestible indispensable amino acid reference ratios in the existing literatures is performed according to the FAO's DIAAS evaluation formula. As depicted in FIG. 18, in this example, the comparison between the digestible indispensable amino acid reference ratio evaluation results and the values listed in the literatures (Observed values) is plotted based on the digestible indispensable amino acid reference ratio evaluation results based on the digestibility predicted values (Predicted values) and the digestibility measured values using a living body (Measured values). Thus, this example indicates that the digestible indispensable amino acid reference ratio can be evaluated accurately using the present digestibility prediction technique. SAA and AAA depicted in FIG. 18 mean sulfur-containing amino acids (sulphur amino acids (methionine + cysteine)) and aromatic amino acids (aromatic amino acids (phenylalanine + tyrosine)), respectively.

### Example 2

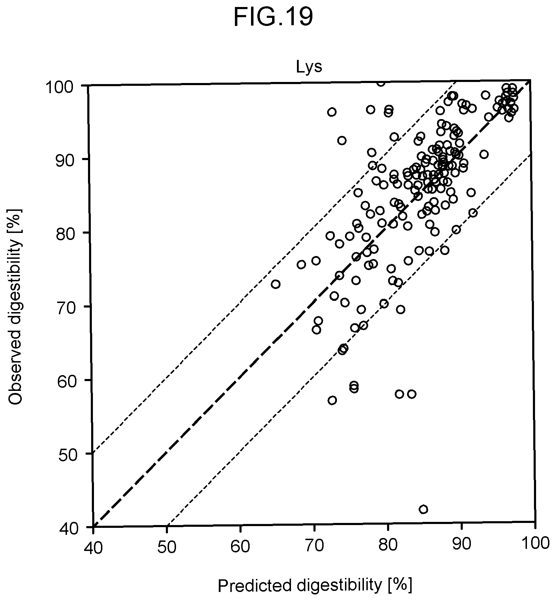

Referring to FIG. 19 to FIG. 22, Example 2 will be explained. FIG. 19 to FIG. 22 are charts of examples of the prediction accuracy verification results according to the present embodiment.

In this example, the digestibility prediction accuracy is verified for a plurality of machine learning models.

Specifically, in this example, the verification of prediction accuracy is performed for various protein food ingredients, including animal proteins and vegetable proteins, using the digestibility data (lysine) as an objective variable, and the amino acid composition, the cooking/processing method, and the type of protein as explanatory variables.

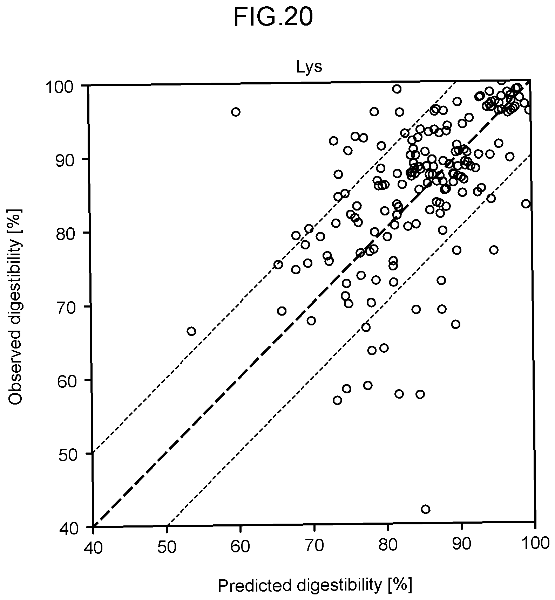

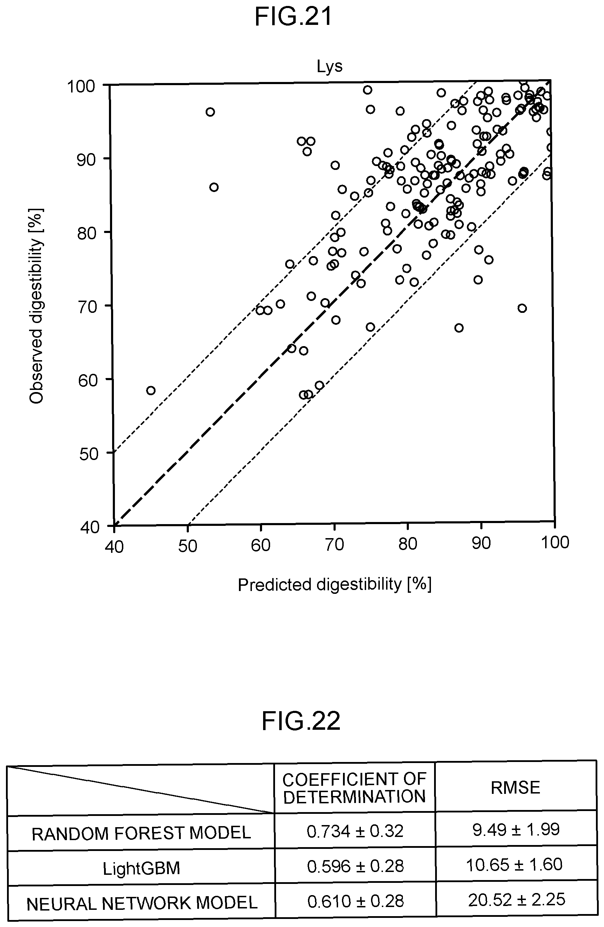

In this example, the prediction accuracy verification results by random forest are obtained as depicted in FIG. 19, the prediction accuracy verification results by LightGBM are obtained as depicted in FIG. 20, and the prediction accuracy verification results by a neural network are obtained as depicted in FIG. 21.

As depicted in FIG. 22, in this example, the means and the standard errors of the coefficient of determination and the root mean square error (RMSE) in predicting digestibility of His, Ile, Leu, Lys, Met, Phe, Thr, Trp, and Val were verified. It is shown that all models are capable of predicting digestibility.

### Example 3

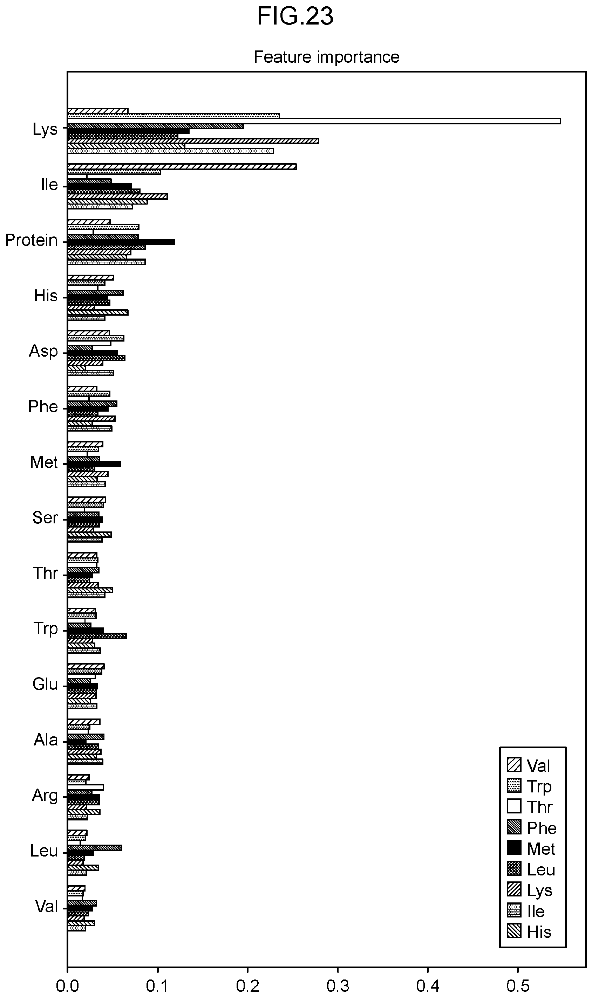

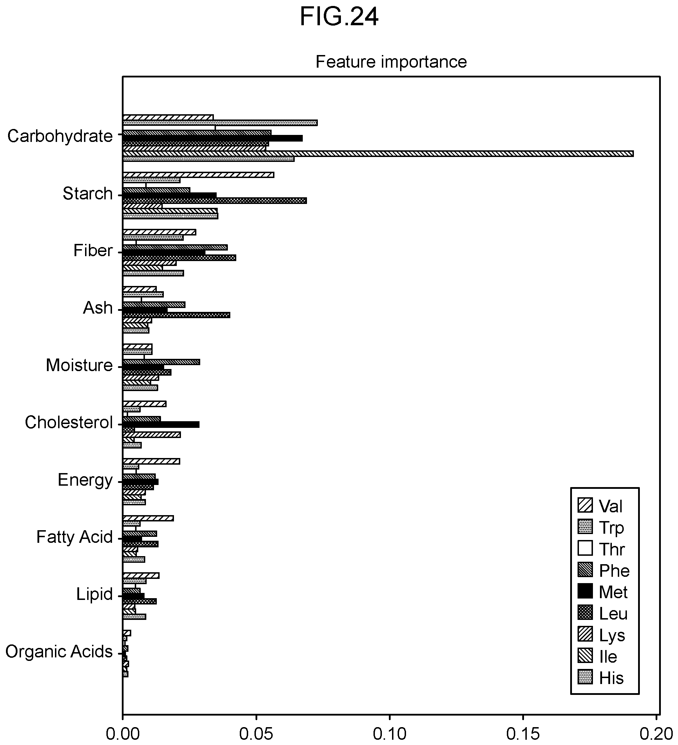

Referring to FIG. 23 and FIG. 24, Example 3 will be explained. FIG. 23 and FIG. 24 are charts of examples of explanatory variable importance analysis results according to the present embodiment.

In this example, nutritional data (Carbohydrate, Starch, Fiber, Ash, Moisture, Cholesterol, Energy, Fatty Acid, Lipid, and Organic Acids) of materials to be evaluated for digestibility are fitted to data on food ingredients most similar to food ingredients listed in Standard Tables of Food Composition in Japan -2020- (eighth revised edition) and used as explanatory variables in the digestibility prediction model.

In this example, 14 types of amino acid compositions of the materials to be evaluated (Arg, His, Ile, Leu, Lys, Met, Phe, Thr, Trp, Val, Ala, Asp, Glu, and Ser) and the crude protein content of the materials to be evaluated are added to explanatory variables (the cooking/processing method and the type of protein are not added to explanatory variables).

In this example, a random forest model for predicting digestibility is constructed using the above explanatory variables, and feature importance analysis is performed on the explanatory variables.

As depicted in FIG. 23 and FIG. 24, this example indicates a possibility that, among the nutritional data other than amino acid composition and crude protein content data, carbohydrate, starch, and dietary fiber contents (FIG. 24) in the materials to be evaluated may contribute to digestibility prediction.

The graphs in FIG. 23 and FIG. 24 indicate the results of the model in which Val (first from the top), Trp (second from the top), Thr (third from the top), Phe (fourth from the top), Met (fifth from the top), Leu (sixth from the top), Lys (seventh from the top), Ile (eighth from the top), and His (ninth from the top) are used as the objective variables.

Based on these results, this example indicates that the nutritional data other than amino acid composition can explain the features related to the digestibility of the materials to be evaluated and can be used for prediction.

### Example 4

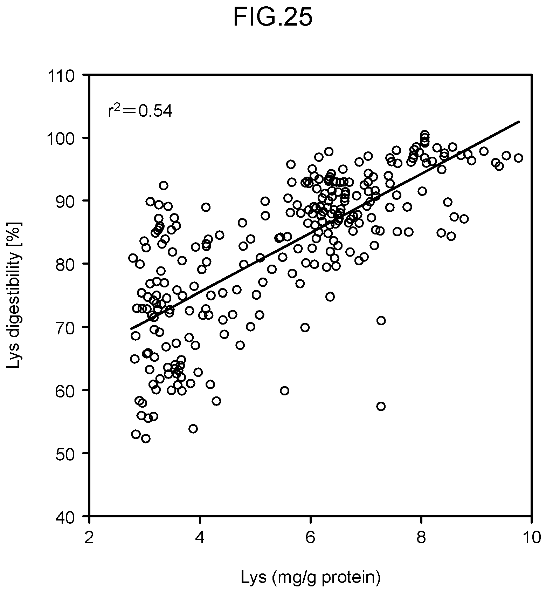

Referring to FIG. 25 to FIG. 27, Example 4 will be explained. FIG. 25 to FIG. 27 are charts of examples of correlation according to the present embodiment.

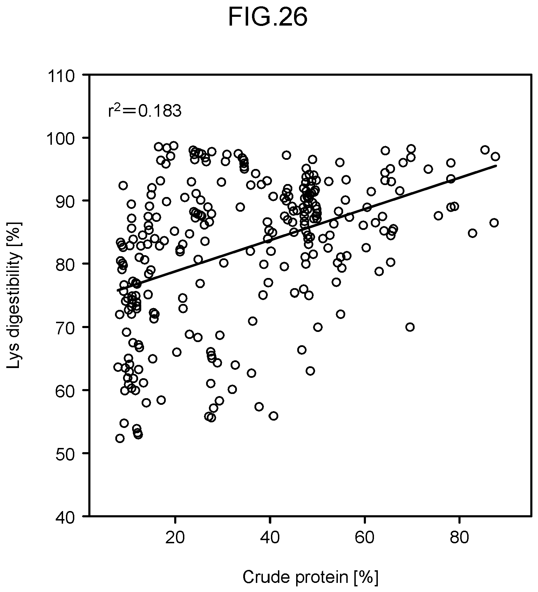

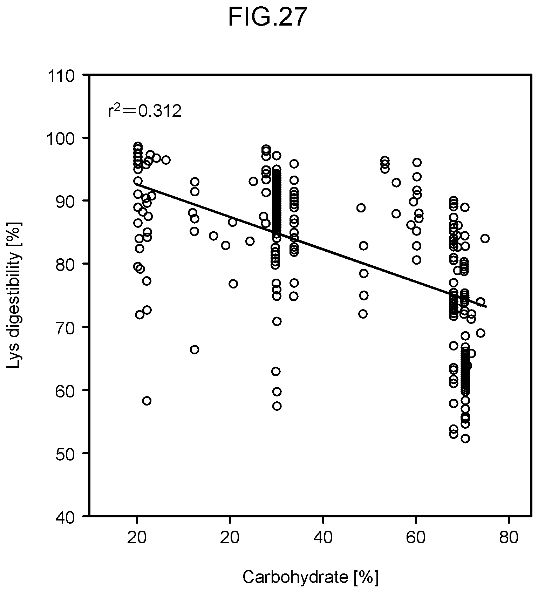

In this example, the correlations of various protein ingredients to be evaluated are analyzed. The correlation between digestibility (lysine) and lysine content is analyzed as depicted in FIG. 25. The correlation between digestibility (lysine) and crude protein content is analyzed as depicted in FIG. 26. The correlation between digestibility (lysine) and carbohydrate content is analyzed as depicted in FIG. 27.

As a result, as depicted in FIG. 25 and FIG. 26, a positive correlation is found between digestibility (lysine) and lysine content and between digestibility (lysine) and crude protein content, while a negative correlation is found between digestibility (lysine) and carbohydrate content.

Thus, it can be confirmed that as the protein content increases, the reaction between the protein ingredient and digestive enzymes becomes easier and therefore the digestibility of the protein ingredient increases, whereas as certain impurities such as carbohydrates increase, the accessibility of digestive enzymes to the protein ingredient decreases and therefore the digestibility of the protein ingredient decreases.

### 4. Other Embodiments

In addition to the embodiment, the present invention may be implemented in a variety of different embodiments within the scope of the technical concept described in the claims.

For example, among the pieces of processing explained in the embodiment, all, some, or one of the pieces of processing explained as being performed automatically can be performed manually, or all, some, or one of the pieces of processing explained as being performed manually can be performed automatically in a known manner.

The processing procedures, control procedures, specific names, information including parameters such as registration data and search conditions for each processing, screen examples, and database configuration described in the present description and in the drawings may be changed as desired unless otherwise specified.

Each of the components depicted in the drawings for the food evaluating apparatus 100 and the like is a functional concept and is not necessarily physically configured as depicted in the drawings.

For example, all, some, or any one of the processing functions of the food evaluating apparatus 100 and the like, especially the processing functions performed by the control unit, may be achieved by a CPU and a computer program interpreted and executed by the CPU, or may be achieved as hardware using wired logic. The computer program is recorded on a non-transitory computer-readable recording medium including programmed instructions to cause an information processing apparatus to perform the processing described in the present embodiment, and is read mechanically as needed. In other words, a computer program is recorded in a storage unit such as a ROM or a hard disk drive (HDD) to give instructions to the CPU in cooperation with an OS to perform various processing. This computer program is executed by being loaded into a RAM and constitutes a control unit in cooperation with the CPU.

The computer program may be stored in an application program server connected via any network 300 to the food evaluating apparatus 100 and the like and may be downloaded in whole or in part as needed.

The computer program for performing the processing described in the present embodiment may be stored in a non-transitory computer-readable recording medium, or may be configured as a program product. As used herein, "recording medium" includes any "portable physical medium" such as a memory card, a universal serial bus (USB) memory, a secure digital (SD) card, a flexible disk, a magneto-optical disk, a ROM, an erasable programmable read only memory (EPROM), an electrically erasable and programmable read only memory (EEPROM (registered trademark)), a compact disc read only memory (CD-ROM), a magneto optical disk (MO), a digital versatile disc (DVD), and a Blu-ray (registered trademark) disc.

The "computer program" is a data processing method written in any language or description method, and can be in any format such as a source code or a binary code. The "computer program" is not necessarily limited to those configured singly, and includes those configured in a distributed manner as multiple modules or libraries and those that cooperate with a separate computer program such as an OS to achieve their functions. Any well-known configuration and procedures can be used as a specific configuration and reading procedure for reading a recording medium in each apparatus described in the present embodiment, an installation procedure after reading, and the like.

Various databases and the like stored in the storage unit are storage units such as memory devices such as RAM and ROM, fixed disk devices such as hard disks, flexible disks, and optical discs and store various programs, tables, databases, files for web pages, and the like used for various processing and website provision.

The food evaluating apparatus 100 and the like may be configured as an information processing apparatus such as a known personal computer or workstation, or may be configured as the information processing apparatus connected to any peripheral device. The food evaluating apparatus 100 and the like may be achieved by implementing software (including a computer program or data) to cause the apparatus to achieve the processing described in the present embodiment.

A specific form of distribution and integration of the apparatus is not limited to that depicted in the drawings. The apparatus can be configured by functionally or physically distributing or integrating all or part of the apparatus in any units according to various additions or according to functional loads. In other words, the embodiments may be implemented in any combination, or the embodiments may be implemented selectively.

### INDUSTRIAL APPLICABILITY

The present invention is useful in a variety of industries including the food industry and the information processing industry.

### EXPLANATIONS OF LETTERS OR NUMERALS

- 100: food evaluating apparatus

- 102: control unit
- 102a: model constructing unit
- 102b: evaluation target setting unit
- 102c: prediction result obtaining unit
- 102d: correcting unit
- 106: storage unit
- 106a: protein database
- 112: input/output unit
- 300: network

## Claims

1. A food evaluating apparatus comprising:
a storage unit, and a control unit, wherein
the storage unit includes:
a protein storage unit that stores a machine learning model in which an amino acid composition of protein is an explanatory variable and protein digestibility is an objective variable, and
the control unit includes:
an evaluation target setting unit that sets the protein as an evaluation target; and
a prediction result obtaining unit that obtains a digestibility prediction result on the protein digestibility of the evaluation target, using the machine learning model.

2. The food evaluating apparatus according to claim 1, wherein the explanatory variable includes a type of the protein.

3. The food evaluating apparatus according to claim 2, wherein the type of the protein is a taxonomic rank of family, genus, or species of an animal or a plant.

4. The food evaluating apparatus according to any one of claims 1 to 3, wherein the explanatory variable further includes at least one of type of a processing and cooking method, temperature, and heating time.

5. The food evaluating apparatus according to claim 3, wherein the explanatory variable further includes any one, some, or all of formulation data including any one or both of moisture content and hardness, physical-chemical property including any one or both of solubility and emulsifying power, nutrient content including any one, some, or all of dietary fiber content, fat content, carbohydrate content, and mineral content, part data of any one or both of a plant and an animal, and source data of any one, some, or all of country, region, and manufacturer.

6. The food evaluating apparatus according to claim 3, wherein the protein is a protein as a cooking material or a protein contained in a single dish.

7. The food evaluating apparatus according to claim 1, wherein the prediction result obtaining unit further calculates a digestible amino acid content using the digestibility prediction result, the digestible amino acid content being a value obtained by multiplying a content of each amino acid by the protein digestibility.

8. The food evaluating apparatus according to claim 1, wherein the prediction result obtaining unit further calculates a digestible indispensable amino acid content, a digestible indispensable amino acid reference ratio, or a digestible indispensable amino acid score, using the digestibility prediction result, the digestible indispensable amino acid content being a value obtained by multiplying a content of each indispensable amino acid by the protein digestibility, the digestible indispensable amino acid reference ratio being a value obtained by dividing the digestible indispensable amino acid content by a reference value, the digestible indispensable amino acid score being a lowest value of the digestible indispensable amino acid content reference ratio.

9. The food evaluating apparatus according to claim 6, wherein the control unit further includes:
a correcting unit that corrects the digestibility prediction result on the protein digestibility of protein contained in the single dish as the evaluation target, based on a digestibility change due to a combination of food ingredients that constitute the single dish.

10. The food evaluating apparatus according to claim 6, wherein the control unit further includes:
a correcting unit that corrects the digestibility prediction result on the protein digestibility of protein contained in the single dish as the evaluation target, based on a digestibility change due to a combination with component dishes that constitute a menu that includes the single dish.

11. The food evaluating apparatus according to claim 1 or 2, wherein the control unit further includes:
a correcting unit that corrects the digestibility prediction result on the protein digestibility of the evaluation target, based on a digestibility change due to exercise before and after intake of the evaluation target.

12. The food evaluating apparatus according to claim 1 or 2, wherein the control unit further includes:
a correcting unit that corrects the digestibility prediction result on the protein digestibility of the evaluation target, based on a digestibility change due to a constitution of a human who consumes the evaluation target.

13. The food evaluating apparatus according to claim 1 or 2, wherein the prediction result obtaining unit further obtains dietary suggestion data, based on the digestibility prediction result and nutrient data including a dietary reference intake.

14. The food evaluating apparatus according to claim 1 or 2, wherein the prediction result obtaining unit further displays the digestibility prediction result.

15. The food evaluating apparatus according to claim 6, wherein the evaluation target is the protein consumed over a predetermined period of time for one or more meals.

16. The food evaluating apparatus according to claim 13, wherein the prediction result obtaining unit obtains the dietary suggestion data, based on the digestibility prediction result, and the nutrient data including the dietary reference intake according to any one or both of an attribute and an intake purpose of a human who consumes the evaluation target.

17. The food evaluating apparatus according to claim 1, wherein the prediction result obtaining unit further creates product development data for a product that reflects the protein digestibility of the evaluation target, based on the digestibility prediction result.

18. The food evaluating apparatus according to claim 1, wherein the machine learning model is a model including at least one of random forests, neural networks, XGBoost, LightGBM, support vector regression, and linear regression including ElasticNet.

19. A food evaluating apparatus comprising:
a storage unit, and a control unit, wherein
the storage unit includes:
a protein storage unit that stores a machine learning model in which an amino acid composition of protein is an explanatory variable, and a digestible amino acid content, a digestible indispensable amino acid content, a digestible indispensable amino acid reference ratio, or a digestible indispensable amino acid score is an objective variable, and
the control unit includes:
an evaluation target setting unit that sets the protein as an evaluation target; and
a prediction result obtaining unit that obtains the digestible amino acid content, the digestible indispensable amino acid content, the digestible indispensable amino acid reference ratio, or the digestible indispensable amino acid score of the evaluation target, using the machine learning model.

20. A food evaluating apparatus comprising:
a storage unit, and a control unit, wherein
the storage unit includes:
a protein storage unit that stores, as training data, a data set in which at least one of an amino acid composition of protein, a type of the protein, formulation data including any one or both of water content and hardness, physical-chemical property including any one or both of solubility and emulsifying power, a nutrient content including any one, some, or all of dietary fiber content, fat content, carbohydrate content, and mineral content, part data of any one or both of a plant and an animal, source data of any one, some, or all of country, region, and manufacturer, a type of a processing and cooking method, a cooking temperature, and a cooking time, and an in vitro analysis result on any one or both of enzyme activity inhibition and digestibility is an explanatory variable, and protein digestibility in an in vitro process or an in vivo process is an objective variable, and
the control unit includes:
a model constructing unit that trains a machine learning model with the training data.

21. A food evaluating method executed by a food evaluating apparatus including:
a storage unit, and a control unit, wherein
the storage unit includes:
a protein storage unit that stores a machine learning model in which an amino acid composition of protein is an explanatory variable and protein digestibility is an objective variable,
the method executed by the control unit comprising:
an evaluation target setting step of setting the protein as an evaluation target; and
a prediction result obtaining step of obtaining a digestibility prediction result on the protein digestibility of the evaluation target, using the machine learning model.

22. A food evaluating method executed by a food evaluating apparatus including:
a storage unit, and a control unit, wherein
the storage unit includes:
a protein storage unit that stores, as training data, a data set in which at least one of an amino acid composition of protein, a type of the protein, formulation data including any one or both of water content and hardness, physical-chemical property including any one or both of solubility and emulsifying power, a nutrient content including any one, some, or all of dietary fiber content, fat content, carbohydrate content, and mineral content, part data of any one or both of a plant and an animal, source data of any one, some, or all of country, region, and manufacturer, a type of a processing and cooking method, a cooking temperature, and a cooking time, and an in vitro analysis result on any one or both of enzyme activity inhibition and digestibility is an explanatory variable, and protein digestibility in an in vitro process or an in vivo process is an objective variable,
the method executed by the control unit comprising:
a model constructing step of training a machine learning model with the training data.

23. A food evaluating program including programmed instructions for causing, when executed by a food evaluating apparatus including a storage unit that includes a protein storage unit that stores a machine learning model in which an amino acid composition of protein is an explanatory variable and protein digestibility is an objective variable, and a control unit,
the control unit of the food evaluating apparatus to perform a food evaluating method comprising:
an evaluation target setting step of setting the protein as an evaluation target; and
a prediction result obtaining step of obtaining a digestibility prediction result on the protein digestibility of the evaluation target, using the machine learning model.
